# EUROPÄISCHE PATENTSCHRIFT

(11) **EP 1 492 546 B1**
(45) Veröffentlichungstag und Bekanntmachung des Hinweises auf die Patenterteilung: **02.05.2007**
(21) Anmeldenummer: 03727197.0
(22) Anmeldetag: 10.04.2003
(51) Int. Cl.: A61K 31/70, A61P 25/02

(54) **VERWENDUNG VON PYRIMIDINNUKLEOTIDEN ZUR BEHANDLUNG VON SCHAEDIGUNGEN DES PERIPHEREN NERVENSYSTEMS**
USE OF PYRIMIDINE NUCLEOTIDES FOR THE TREATMENT OF AFFECTIONS OF THE PERIPHERAL NERVOUS SYSTEM
UTILISATION DE NUCLEOTIDES DE PYRIMIDINE POUR TRAITER DES DOMMAGES DU SYSTEME NERVEUX PERIPHERIQUE

(30) Priorität: 10.04.2002 DE 10215753
(43) Veröffentlichungstag der Anmeldung: 05.01.2005
(73) Patentinhaber: Trommsdorff GmbH & Co. KG Arzneimittel, 52475 Alsdorf (DE)
(72) Erfinder: HEDDING-ECKERICH, Monika, 41564 Kaarst (DE)
(74) Vertreter: Arth, Hans-Lothar
(86) Internationale Anmeldenummer: PCT/DE2003/001203
(87) Internationale Veröffentlichungsnummer: WO 2003/086417

(56) Entgegenhaltungen:
- DE-A- 4 204 108
- WATTIG B ET AL: "NUCLEOTIDES ACCELERATE REGENERATION OF NERVES" ZEITSCHRIFT FUER KLINISCHE MEDIZIN (BERLIN), Bd. 46, Nr. 19, 1991, Seiten 1371-1373, XP009014725 ISSN: 0233-1608
- WATTIG B ET AL: "[Acceleration of muscle regeneration by nucleotide administration. Experimental morphometric studies]" ZENTRALBLATT FUR PATHOLOGIE. GERMANY 1991, Bd. 137, Nr. 5, 1991, Seiten 409-413, XP009014728 ISSN: 0863-4106
- WATTIG B ET AL: "Acceleration of nerve and muscle regeneration by administration of nucleotides--electroneurophysiological and morphometrical investigations." ACTA HISTOCHEMICA. SUPPLEMENTBAND. GERMANY 1992, Bd. 42, 1992, Seiten 333-339, XP009014730 ISSN: 0567-7556
- KRETSCHMAR C ET AL: "[Medicamentous therapy of alcoholic polyneuropathy. Randomized double-blind study comparing 2 vitamin B preparations and a nucleotide preparation]" FORTSCHRITTE DER MEDIZIN. GERMANY 20 NOV 1996, Bd. 114, Nr. 32, 20. November 1996 (1996-11-20), Seiten 439-443, XP009014727 ISSN: 0015-8178

## Beschreibung

Die Erfindung betrifft die Verwendung von Uridin-5'-monophosphat oder Cytidin-5'-monophosphat zur Herstellung einer pharmazeutischen Zusammensetzung zur Behandlung von Schädigungen des peripheren Nervensystems, insbesondere von Polyneuropathien, Neuritiden und Myopathien, sowie die Verwendung zur Stimulation der Nervenregeneration.

Nervenverletzungen sind Ereignisse, die in der Traumatologie an erster Stelle stehen aber auch im Gefolge entzündlich-, metabolisch-, endokrin-, vaskulär- und toxisch-bedingter Erkrankungen auftreten.

Wissenschaftliche Erkenntnisse lassen vermuten, daß Axone für die Funktionsfähigkeit von peripheren Nerven von Bedeutung sind, da sie für die Transport- und Leitfähigkeit von Versorgungssubstanzen und Informationen wichtig zu sein scheinen.

Kommt es zu einer Nervenschädigung mit Durchtrennung von Nervenbahnen, so setzt im Läsionsbereich eine Aussprossung von Axonen ein, und im Nerv läßt sich eine Form eines eigenen Reparatursystems erkennen, das zur Regeneration von Nerven etwa im Fall der Polyneuropathie führt.

Experimentell wurden drei Phasen beim Reparatursystem erkannt.
1. Eine Latenzphase vom 1. bis 2. Tag nach dem Trauma.
   Das Axon degeneriert an der Läsionsstelle. Es kommt zur Fragmentierung. Auch das Myelin des Hüllgewebes wird von diesem Prozeß erfaßt und zerfällt innerhalb von etwa 8 Tagen.
2. Eine Regenerationsphase vom 2. bis 12. Tag.
   Am proximalen und distalen Stumpf des durchtrennten Nervenbereichs teilen sich die Schwannschen Zellen. Ein junges Axon sproßt aus und akkumuliert Proteine, Lipide und RNA. Büngnersche Bänder bemühen sich, die Verbindung zwischen den durchtrennten Faserstücken wieder aufzubauen. An dieser Leitstruktur wandert das junge Axon entlang.
3. Eine Reifungsphase vom 12. bis 90. Tag.
   Die neue Nervenfaser nimmt infolge der Myelinisierung an Dicke zu. Neue Zellstrukturen bilden sich.

Damit dieses Reparatursystem funktioniert, bedarf es bestimmter Substrate wie Cytidin und Uridin, welche als Energiespender wirken. Nukleinsäuren sind die Basis der Nervenregeneration. Nukleoside werden von den Nervenzellen aufgenommen und zu Nukleotiden umgesetzt. Nukleotide gelangen in die Axone, die maßgeblich am Regenerationsprozeß beteiligt sind. Cytidin und Uridin bewirken dabei die Neusynthese von Strukturbestandteilen der Nervenzelle (J. Cervos-Navarro, Ärzte Zeitung, 1992, Nr. 131, S. 2).

In klinischen Studien an Patienten mit einer Polyneuropathie führte die externe Applikation eines Gemisches aus Cytidin und Uridin zu einer Besserung der typischen Polyneuropathiesymptome.

Eine Studie an Wistarratten belegte die gute Wirksamkeit der Wirkstoffkombination von Uridinmonophosphat (UMP) und Cytidinmonophosphat (CMP) in bezug auf die Regeneration traumatisch geschädigter peripherer Nerven.

In dieser Versuchsreihe wurde einer Gruppe von Versuchstieren UMP, einer anderen CMP und einer weiteren ein Gemisch aus UMP und CMP verabreicht. Nur die Gruppe, welche die Wirkstoffkombination aus UMP und CMP erhalten hatte, zeigte eine vergrößerte Faserfläche auf der Basis erweiterter Myelinscheidenflächen. Die Strukturanalyse der Nervenfasern belegte, daß in der UMP/CMP-Gruppe eine merkliche Vergrößerung der mittleren Faserfläche eingetreten war, die durch vergrößerte Myelin- und Axonflächen bedingt war.

Die Versuchstiere, welche nur die Einzelsubstanzen UMP oder CMP erhalten hatten, zeigten keine vergleichbaren Resultate (B Wattig et al., Zeitschrift für klinische Medizin, 1991, 46,1371-1373).

Der vorliegenden Erfindung liegt die Aufgabe zugrunde physiologisch gut verträgliche Substanzen bereitzustellen, welche zur Behandlung von Schädigungen des peripheren Nervensystems sowie zur Stimulation der Nervenregeneration eingesetzt werden können.

Diese Aufgabe wird durch die technische Lehre des unabhängigen Anspruchs 1 gelöst. Weitere vorteilhafte Ausgestaltungen, Aspekte und Details der Erfindung ergeben sich aus den abhängigen Ansprüchen, der Beschreibung und den Beispielen.

Überraschenderweise wurde gefunden, daß es zur Behandlung von Schädigungen des peripheren Nervensystems sowie zur Stimulation der Nervenregeneration nicht einer Wirkstoffkombination von mindestens 2 Pyrimidinnukleotiden bedarf, sondern die Einzelsubstanzen Uridin-5'-monophosphat oder Cytidin-5'-monophosphat eine hervorragende Wirkung zeigen.

Entgegen den oben erwähnten Erkenntnissen aus der Tierversuchsstudie, welche den Einzelsubstanzen UMP und CMP eine Wirksamkeit bei der Behandlung von Schädigungen des peripheren Nervensystems bzw. der Nervenregeneration absprechen, konnte nachgewiesen werden, daß UMP als auch CMP eine hohe Wirksamkeit bei dieser Indikation aufweisen.

Somit betrifft die Erfindung die Verwendung von Uridin-5'-monophosphat oder Cytidin-5'-monophosphat zur Herstellung einer pharmazeutischen Zusammensetzung zur Behandlung von Schädigungen des peripheren Nervensystems und/oder zur Stimulation der Nervenregeneration. Als Beispiele für Schädigungen des peripheren Nervensystems gelten beispielsweise Polyneuropathien, Neuritiden und Myopathien.

Bevorzugte Indikationen sind degenerative Erkrankungen der Wirbelsäule, diabetische Polyneuropathien, Polyneuropathien nach Alkoholabusus, andere toxische Polyneuropathien, Facialisparese, Gesichtsneuralgien, multiple Sklerose, Wurzelneuritiden, Zervikalsyndrom, Schulter-Arm-Syndrom, Ischialgie, Lumbago, Interkostalneuralgie, Trigeminusneuralgie sowie Herpes zoster.

Bei dem erfindungsmäßigen Einsatz ist UMP dem CMP noch vorzuziehen.

Das Pyrimidinnukleotid UMP oder CMP wird in einer täglichen Dosis von 1 - 100 mg, bevorzugt von 5 - 50 mg, weiterhin bevorzugt 7 bis 40 mg und insbesondere bevorzugt von 10 bis 35 mg eingesetzt.

Eine weitere Verwendung von UMP oder CMP besteht in der Herstellung einer pharmazeutischen Zusammensetzung, welche zur Behandlung von Schädigungen des peripheren Nervensystems und/oder zur Stimulation der Nervenregeneration geeignet ist.

Derartige pharmazeutische Zusammensetzungen können neben dem Pyrimidinnukleotid die üblichen festen oder flüssigen Träger, Verdünnungs- oder Lösungsmittel bzw. die üblicherweise verwendeten pharmazeutischen Hilfsstoffe enthalten. Die pharmazeutischen Zusammensetzungen werden entsprechend der gewünschten Applikationsart bevorzugt mit einer Wirkstoffkonzentration an Pyrimidinnukleotid von 1 - 100 mg, vorzugsweise 5 - 50 mg, weiterhin bevorzugt 7 - 40 mg und insbesondere bevorzugt 10 - 35 mg in bekannter Weise hergestellt.

Die erfindungsgemäß einsetzbaren Pyrimidinnukleotide sowie die erfindungsgemäß einsetzbaren pharmazeutischen Zusammensetzungen eignen sich für eine intravenöse, intraperitoneale, intramuskuläre, subkutane, rektale, vaginale, transdermale, topische, intradermale, intestinale, orale, intragastrale, intrakutane, intranasale, intrabuccale, perkutane, sublinguale oder irgend eine andere Applikation.

Die bevorzugten pharmazeutischen Zusammensetzungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen, Dispersionen, Suspensionen, Depotformen oder Inhalationslösungen.

Entsprechende Tabletten können beispielsweise durch Mischen eines Pyrimidinnukleotids mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelantine, Gleitmitteln wie Magnesiumstearat oder Talk und/oder Mitteln zur Erzielung eines Depoteffektes wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäß einsetzbaren Wirkstoff können zusätzlich geschmacksverbessernde Mittel wie Saccharin, Cyclamat oder Zucker sowie Aromastoffe wie Vanillin oder Orangenextrakt enthalten. Sie können außerdem Suspendierhilfsstoffe wie Natriumcarboxymethylcellulose oder Konservierungsmittel wie p-Hydroxybenzoate enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Eine bevorzugte Zusammensetzung enthält beispielsweise Natriumcitrat-Dihydrat, wasserfreie Citronensäure, Magnesiumstearat, hochdisperses Siliciumdioxid, Mannitol, Gelatine und gegebenenfalls Farbstoffe.

Geeignete Suppositorien lassen sich beispielsweise durch Vermischen mit dafür vorgesehenen Trägermitteln wie Neutralfetten oder Polyethylenglykol bzw. Derivaten davon herstellen.

Selbstverständlich kommen auch parenterale Zubereitungen, wie Injektions- oder Infusionslösungen in Betracht. Für die parenterale Applikation eignen sich insbesondere Injektionslösungen eines Pyrimidinnukleotids in physiologischer Kochsalzlösung.

Verfahren zur Herstellung diverser Formulierungen sowie die verschiedenen Applikationsmethoden sind dem Fachmann bekannt und beispielsweise in "Remington's Pharmaceutical Sciences, Mack Publishing Co., Easton PA" eingehend beschrieben.

### Beispiele

Im folgenden wird am Beispiel von Uridin-5'-monophosphate (UMP) die Wirksamkeit von Pyrimidinnukleotiden zur Behandlung von Schädigungen des peripheren Nervensystems sowie zur Stimulation der Nervenregeneration am Beispiel der Indikation Lumbago beschrieben.

80 weibliche als auch männliche Patienten im Alter zwischen 20 und 63 Jahren wurden nach dem Zufallsprinzip in zwei Gruppen aufgeteilt, von denen eine einer Behandlung mit UMP und die andere einer Placebo-Behandlung unterzogen wurde.

Die Behandlung erstreckte sich über vier aufeinanderfolgende Tage, wobei die Behandlung auch vorzeitig abgebrochen werden konnte, sobald sich die Symptome derart gebessert hatten, daß eine weitere Behandlung überflüssig wurde.

Appliziert wurde einmal täglich in eine Armvene eine Injektionslösung von 19,349 mg UMP (dies entspricht einer Masse von 12,834 mg Uridin) in 250 ml physiologischer Kochsalzlösung über einen Zeitraum von 30 Minuten. Die Vergleichsgruppe erhielt als Placebo nur die 250 ml physiologische Kochsalzlösung ohne einen Wirkstoff.

Für diese klinische Studie wurden nur Patienten mit einer Schmerzintensität von mindestens 40 mm auf der VAS-Skala (VAS: visual analogue scale) ausgewählt.

Die Schmerzintensität wird auf der linearen VAS-Skala in der Einheit "mm" angegeben und wurde bestimmt, indem die Patienten dazu aufgefordert wurden, auf der linearen VAS-Skala den Punkt zu markieren, der ihrer jeweiligen Schmerzintensität entsprach. Dabei wird kein spürbarer Schmerz mit dem Wert 0 mm und der größtmögliche Schmerz mit dem Wert 100 mm angegeben.

Die Schmerzintensität der Patienten wurde kontinuierlich überprüft, wobei die Patienten auch die jeweilige Dauer der einzelnen Schmerzperioden anzugeben hatten.

### Ergebnisse

Bereits innerhalb der ersten 24h nach der ersten Injektion kann ein deutlicher Rückgang der Schmerzen bei den UMP-Patienten gegenüber den Patienten, die nur ein Placebo erhalten hatten, festgestellt werden (s. Tabelle I).

Tabelle I belegt, daß bereits nach kurzer Zeit nach der ersten Injektion die Schmerzintensität im Ruhe- und Bewegungszustand bei der UMP-Patientengruppe stark abnimmt. Der Finger-Boden-Abstand stellt ein Maß für die Beweglichkeit, d.h. für den Grad der Fähigkeit, den Rücken zu beugen, dar und wird in der Einheit "cm" angegeben, wobei der Abstand zwischen der Fingerspitze bei ausgestreckten Armen und dem Bogen bei maximal möglicher Beugung des Rückens gemessen wurde.

**Tabelle I: Durchschnittswerte der UMP- und Placebo-Patientengruppe**

| | **UMP** | | **Placebo** | |
|---|---|---|---|---|
| | Basislinie | 24h nach Inj. | Basislinie | 24h nach Inj. |
| Schmerz im Ruhezustand (mm) | 60.3 | 46.2 | 63.5 | 53.6 |
| Schmerz in Bewegung (mm) | 76.1 | 56.9 | 76.3 | 65.1 |
| Tägliche Schmerzdauer (h) | 15.8 | 10.7 | 14.8 | 11.7 |
| Vorhandensein von Myogelose | 100% | 90% | 100% | 100% |
| Finger-Boden-Abstand (cm) | 43.8 | 32.5 | 46.8 | 38.3 |

Tabelle II stellt die Unterschiede im Rückgang der Schmerzintensität im Vergleich von UMP- und Placebo-Patientengruppe heraus.

**Tabelle II: Schmerzrückgang bei UMP- und Placebo-Patientengruppe**

| | Δ **UMP** | Δ **Placebo** | Δ UMP - Δ Placebo |
|---|---|---|---|
| Schmerz im Ruhezustand (mm) | -12.8 | -9.4 | -3.4 |
| Schmerz in Bewegung (mm) | -17.9 | -11.1 | -6.8 |
| Tägliche Schmerzdauer (h) | -4.3 | -2.9 | -1.4 |
| Finger-Boden-Abstand (cm) | -10.1 | -7.4 | -2.7 |

Tabelle II belegt den deutlich besseren Schmerzrückgang bei der UMP-Patientengruppe im Vergleich mit der Placebo-Patientengruppe. Im Verlauf der weiteren drei Tage konnte festgestellt werden, daß sich die Differenzen für den Schmerzrückgang, sowie für die tägliche Schmerzdauer als auch für den Finger-Boden-Abstand der UMP-Patientengruppe (Δ UMP) langsam den Differenzen der Placebo-Patientengruppe (Δ Placebo) angenähert haben, wobei jedoch stets die Absolutwerte der oben genannten Testparameter bei der UMP-Patientengruppe unter den entsprechenden Absolutwerten für die Placebo-Patientengruppe lagen.

Somit belegt die durchgeführte klinische Studie eindeutig die pharmakologische Wirksamkeit eines einzeln verabreichten Pyrimidinnukleotids und die bisher in der Fachliteratur vertretene Ansicht, daß nur Kombinationen von Pyrimidinnukleotiden eine angemessene Wirkung erzielen, ist damit widerlegt.

## Patentansprüche

1. Verwendung von Uridin-5'-monophosphat oder Cytidin-5'-monophosphat zur Herstellung einer pharmazeutischen Zusammensetzung zur Behandlung von Schädigungen des peripheren Nervensystems und/oder zur Stimulation der Nervenregeneration.

2. Verwendung gemäß Anspruch 1, **dadurch gekennzeichnet, daß** es sich um Uridin-5'-monophosphat handelt.

3. Verwendung gemäß Anspruch 1 oder 2, **dadurch gekennzeichnet, daß** es sich bei den Schädigungen des peripheren Nervensystems um Polyneuropathien, Neuritiden und/oder Myopathien handelt.

4. Verwendung gemäß Anspruch 3, **dadurch gekennzeichnet, daß** es sich bei den Polyneuropathien, Neuritiden und Myopathien um degenerative Erkrankungen der Wirbelsäule, diabetische Polyneuropathien, Polyneuropathien nach Alkoholabusus, andere toxische Polyneuropathien, Facialisparese, Gesichtsneuralgien, multiple Sklerose, Wurzelneuritiden, Zervikalsyndrom, Schulter-Arm-Syndrom, lschialgie, Lumbago, Interkostalneuralgie, Trigeminusneuralgie und/oder Herpes zoster handelt.

5. Verwendung gemäß eines der vorhergehenden Ansprüche, **dadurch gekennzeichnet, daß** das Uridin-5'-monophosphat oder Cytidin-5'-monophosphat in einer täglichen Dosis von 1 - 100 mg, bevorzugt von 5 - 50 mg und insbesondere bevorzugt von 7 bis 40 mg eingesetzt wird.

## Claims

1. Use of uridine-5'-monophosphate or cytidine-5'-monophosphate for preparing a pharmaceutical composition for the treatment of disorders of the peripheral nervous system and/or for the stimulation of the nerve regeneration.

2. Use according to claim 1, **characterized in that** uridine-5'-monophosphate is concerned.

3. Use according to claim 1 or 2, **characterized in that** the disorders of the peripheral nervous system concerned are polyneuropathies, neuritides and/or myopathies.

4. Use according to claim 3, **characterized in that** the polyneuropathies, neuritides and myopathies concerned are degenerative diseases of the spinal column, diabetic polyneuropathies, polyneuropathies due to alcohol abuse, other toxic polyneuropathies, facial nerve paresis, facial neuralgias, multiple sclerosis, radiculoneuritides, cervical syndrome, shoulder-hand syndrome, sciatica, lumbago, intercostal neuralgia, trigeminal neuralgia and/or herpes zoster.

5. Use according to any one of the preceding claims, **characterized in that** uridine-5'-monophosphate or cytidine-5'-monophosphate are applied in a daily dose of 1 - 100 mg, preferably of 5 - 50 mg and particularly preferred of 7 to 40 mg.

## Revendications

1. Utilisation de uridine-5'-monophosphate ou de cytidine-5'-monophosphate pour la préparation d'une composition pharmaceutique pour le traitement de lésions du système nerveux périphérique et/ou pour la stimulation de la régénération nerveuse.

2. Utilisation selon la revendication 1, **caractérisée en ce que** l'uridine-5'-monophosphate est concerné.

3. Utilisation selon la revendication 1 ou 2, **caractérisée en ce que** les lésions du système nerveux périphérique concernés sont des polyneuropathies, des névrites et/ou des myopathies.

4. Utilisation selon la revendication 3, **caractérisée en ce que** les polyneuropathies, névrites et myopathies concernées sont des maladies dégénératives au niveau de la colonne vertébrale, des polyneuropathies diabétiques, des polyneuropathies due à l'abus d'alcool, d'autres polyneuropathies toxiques, la paralysie faciale, des névralgies faciales, la sclérose en plaques, les radiculites, le syndrome cervical, le syndrome épaule-main, la sciatique, le lumbago, la névralgie intercostale, la névralgie du trijumeau et/ou l'herpès zoster.

5. Utilisation selon l'une des revendications précédentes, **caractérisée en ce que** l'uridine-5'-monophosphate ou le cytidine-5'-monophosphate sont appliqués à une dose quotidienne de 1 à 100 mg, de manière préférée de 5 à 50 mg et de manière la plus préférée de 7 à 40 mg.
